# EUROPEAN PATENT SPECIFICATION

(11) **EP 1 148 897 B1**
(45) Date of publication and mention of the grant of the patent: **10.12.2003**
(21) Application number: 00905869.4
(22) Date of filing: 31.01.2000
(51) Int. Cl.: A61L 27/22, A61L 27/38, A61K 38/18, C07K 14/51

(54) **METHODS AND COMPOSITIONS FOR HEALING AND REPAIR OF ARTICULAR CARTILAGE**
METHODEN UND ZUSAMMENSETZUNGEN ZUR HEILUNG UND REPARATUR VON GELENKKNORPELN
PROCEDES ET COMPOSITIONS PERMETTANT LA CICATRISATION ET LA REPARATION DU CARTILAGE ARTICULAIRE

(30) Priority: 01.02.1999 US 118160 P; 28.01.2000 US 493543
(43) Date of publication of application: 31.10.2001
(73) Proprietor: Genetics Institute, LLC, Cambridge, MA 02140 (US)
(72) Inventor: ZHANG, Renwen, Edison, NJ 08820 (US); PELUSO, Diane, Marshfield, MA 02050 (US); MORRIS, Elisabeth, Sherborn, MA 01770 (US)
(74) Representative: Wileman, David Francis, Dr.
(86) International application number: US0002430
(87) International publication number: WO00044413

(56) References cited:
- WO-A-94/01557
- WO-A-96/39170
- WO-A-98/31788
- US-A- 5 658 882
- US-A- 5 786 217
- TAKAGI K ET AL: "THE ROLE OF BONE MARROW IN BONE MORPHOGENETIC PROTEIN-INDUCED REPAIR OF FEMORAL MASSIVE DIAPHYSEAL DEFECTS" CLINICAL ORTHOPAEDICS AND RELATED RESEARCH,US,PHILADELPHIA, PA, vol. 171, 1 January 1982 (1982-01-01), pages 224-231, XP002065500
- WANG E A: "BONE MORPHOGENETIC PROTEINS (BMPS): THERAPEUTIC POTENTIAL IN HEALING BONY DEFECTS" TRENDS IN BIOTECHNOLOGY,GB,ELSEVIER PUBLICATIONS, CAMBRIDGE, vol. 11, no. 9, 1 September 1993 (1993-09-01), pages 379-383, XP002065499 ISSN: 0167-7799
- REDDI A H ET AL: "BONE MORPHOGENETIC PROTEINS. POTENTIAL ROLE IN OSTEOPOROSIS" OSTEOPOROSIS,GB,SHEFFIELD, 1 January 1996 (1996-01-01), pages 281-287, XP002065501 ISSN: 1365-3326

## Description

### FIELD OF THE INVENTION

The present invention relates to the field of tissue repair, specifically, the regeneration of stable and functional articular cartilage repair. Thus, the present invention may be useful in reconstructive surgery or other procedures for the regeneration or repair of articular cartilage.

### BACKGROUND OF THE INVENTION

The repair of articular cartilage injuries remains a challenge in present day orthopedics. Several of the current therapeutic strategies are based upon the grafting of chondral and osteochondral tissues. However, despite substantial endeavors in this field, there remains a need for an effective method of repair of articular cartilage defects and injuries.

### SUMMARY OF THE INVENTION

The present invention provides compositions for regenerating functional and physiologically appropriate tissue repair for the repair of articular cartilage injuries and defects. A first aspect of the invention therefore relates to an osteochondral graft for administration to an area in need of articular cartilage regeneration, having applied thereto an effective amount of bone morphogenetic protein (BMP) to stimulate osteogenic activity of infiltrating progenitor cells. The present invention also comprises methods of preparing osteochondral grafts that may be used for treating patients with articular cartilage injuries or defects and in this regard, a second aspect of the invention relates to a process for preparing an osteochondral graft for administration to an area in need of regeneration of articular cartilage, wherein said process comprises applying to the osteochondral graft an effective amount of at least one purified bone morphogenetic protein (BMP) to stimulate osteogenic activity of infiltrating progenitor cells. The methods and compositions of the present invention are advantageous in that they utilize bone morphogenetic proteins (BMPs), which are known to have osteogenic and/or chondrogenic properties, and which may be produced via recombinant DNA technology, and therefore are of potentially unlimited supply. The novel compositions of the present invention are further advantageous in that regeneration of functional articular cartilage may be accelerated or may be of greater ultimate strength and stability, and the tissue formed at the site of the defect or injury is physiologically appropriate.

The use of BMP to augment the repair of articular cartilage defects and injuries may result in better methods for treatment of osteoarthritis, thus obviating, delaying or reducing the need for artificial hip replacements and other common interventions. A third aspect of the invention therefore comprises the use of at least one purified morphogenetic protein (BMP) in the preparation of a medicament for improving integration of an osteochondral graft when administered therewith to an area in need of articular cartilage regeneration. Preclinical evaluations indicate that rhBMP-2 improves early healing of full thickness defects of articular cartilage in rabbits.

### DETAILED DESCRIPTION OF THE INVENTION

According to the present invention, compositions are provided for use in the treatment of patients who suffer from some form of articular cartilage injury or defect. The injury may be the result of acute stress, or injury, such as resulting from participation in athletics, or from accidental occurrences which tear, mar or otherwise injure the articular cartilage.

The compositions are advantageous in that repair or improvement of articular cartilage defects, particularly full thickness articular cartilage defects. Other defects may also be treated by the compositions of the present invention, particularly with an additional procedure in which the site of the defect is further aggravated so as to reach the underlying subchondral bone.

In the present invention, active growth factor, such as a BMP, is added to a suitable tissue source. The tissue source may be an osteochondral graft, either autologous to the patient, or may comprise allograft or artificially prepared tissue. In a preferred embodiment, the tissue source may be chondrocytic: cell cultures, such as chondrocyte or stem cell cultures which have been prepared through ex vivo cell culture methods, with or without additional growth factors. For example, see the disclosure of US5226914; US5811094; US5053050; US5486359; US5786217 and US572333 1.

The tissue may also be harvested by traditional non-cell culture based means, using techniques such as mosaicplasty, in which cartflage is harvested using commercially available instruments such as Acufex7 [Smith and Nephew, Inc., Andover MA]; COR System [Innovasive Technologies, Marlborough MA]; or Arthrex7 Osteochondral Autograft Transfer System [Arthrex, Munich, Germany]. The tissue harvested may be applied directly in the methods of the present invention, or may be combined with the tissue based cell culture systems described above.

### GROWTH FACTOR

The active growth factor used in the present invention is preferably from the subclass of proteins known generally as bone morphogenetic proteins (BMPs), which have been disclosed to have osteogenic, chondrogenic and other growth and differentiation type activities. These BMPs include rhBMP-2, rhBMP-3, rhBMP-4 (also referred to as rhBMP-2B), rhBMP-5, rhBMP-6, rhBMP-7 (rhOP-1), rhBMP-8, rhBMP-9, rhBMP-12, rhBMP-13, rhBMP- 15, rhBMP-16, rhBMP-17, rhBMP-18, rhGDF-1, rhGDF-3, rhGDF-5, rhGDF-6, rhGDF-7, rhGDF-8, rhGDF-9, rhGDF-10, rhGDF-11, rhGDF-12, rhGDF-14. For example, BMP-2, BMP-3, BMP-4, BMP-5, BMP-6 and BMP-7, disclosed in United States Patents 5,108,922; 5,013,649; 5,116,738; 5,106,748; 5,187,076; and 5,141,905; BMP-8, disclosed in PCT publication W091/18098; and BMP-9, disclosed in PCT publication W093/00432, BMP-10, disclosed in United States Patent 5,637,480; BMP-11, disclosed in United States Patent 5,639,638, or BMP-12 or BMP-13, disclosed in United States Patent 5,658,882, BMP-15, disclosed United States Patent 5,635,372 and BMP-16, disclosed in co-pending patent application serial number 08/715,202. Other compositions which may also be useful include Vgr-2, and any of the growth and differentiation factors [GDFs], including those described in PCT applications W094/15965; W094/15949; W095/01801; W095/01802; W094/2168 1; W094/15966; W095/10539; W096/01845; W096/02559 and others. Also useful in the present invention may be BIP, disclosed in W094/01557; HP00269, disclosed in JP Publication number: 7-250688; and MP52, disclosed in PCT application W093/16099. Also useful in the present invention are heterodimers of the above and modified proteins or partial deletion products thereof. These proteins can be used individually or in mixtures of two or more, and rhBMP-2 is preferred.

The BMP may be recombinantly produced, or purified from a protein composition. The BMP may be homodimeric, or may be heterodimeric with other BMPs (e.g., a heterodimer composed of one monomer each of BMP-2 and BMP-6) or with other members of the TGF-β superfamily, such as activins, inhibins and TGF-β1 (e.g., a heterodimer composed of one monomer each of a BNT and a related member of the TGF-β superfamily). Examples of such heterodimeric proteins are described for example in Published PCT Patent Application WO 93/09229. The amount of osteogenic protein useful herein is that amount effective to stimulate increased osteogenic activity of infiltrating progenitor cells, and will depend upon the size and nature of the defect being treated, as well as the carrier being employed. Generally, the amount of protein to be delivered is in a range of from about 0.05 to about 1.5 mg.

In a preferred embodiment, the osteogenic protein is administered together with an effective amount of a protein which is able to induce the formation of tendon- or ligament-like tissue. Such proteins, include BMP-12, BMP-13, and other members of the BMP-12 subfamily, as well as MP52. These proteins and their use for regeneration of tendon and ligament-like tissue are disclosed in United States application serial number serial number 08/362,670, filed on December 22, 1994. In another preferred embodiment, a heterodimer in which one monomer unit is an osteogenic protein such as BMP-2, and the other monomer subunit is a tendon-inducing protein, such as BMP-12, is administered in accordance with the methods described below, in order to induce the formation of a functional attachment between connective tissue and bone.

### APPLICATION OF GROWTH FACTOR

Growth factor may be applied to the tissue source in the form of a buffer solution. One preferred buffer solution is a composition comprising, in addition to the active growth factor, about 1.0 to about 10.0% (w/v) glycine, about 0. 1 to about 5.0% (w/v) of a sugar, preferably sucrose, about 1 to about 20 mM glutamic acid hydrochloride, and optionally about 0.01 to about 0.1% of a nonionic surfactant, such as polysorbate 80. Preferred solutions are from about 1% to about 20% w/v cellulosic carrier/buffer. If desired, a salt may be added.

Other materials which may be suitable for use in application of the growth factors in the compositions of the present invention include hyaluronic acid, surgical mesh or sutures, polyglyconate, temperature-sensitive polymers, demineralized bone, minerals and ceramics, such as calcium phosphates, hydroxyapatite, etc., as well as combinations of the above described materials. In the preferred embodiment of the present invention, however, no carrier is employed.

The growth factor of the present invention, in a suitable buffer such as that described above, or combined with a suitable carrier, may be applied directly to the tissue and/or to the site in need of tissue repair. For example, the growth factor may be physically applied to the tissue through spraying or dipping, or using a brush or other suitable applicator, such as a syringe for injection. Alternatively, or in conjunction, the protein may be directly applied to the site in need of tissue repair.

The following examples further describe the practice of embodiments of the invention with BMP-2. The examples are not limiting, and as will be appreciated by those skilled in the art, can be varied in accordance with the above specification.

### EXAMPLES

### I. Rabbit Allograft

All procedures were carried out with approval from IACUC. Twelve male New Zealand white rabbits (6 months old) were used. Two rabbits served as donors and 10 as recipients. Osteochondral grafts (3.5 mm diameter) were harvested from the trochlear groove or the medial femoral condyle of the donors, and transplanted into a 3.5 mm deep defect in the trochlear groove of the recipient. The graft was bathed in either rhBMP-2 (0.5 mg/ml) or buffer control prior to implantation. The rabbits were sacrificed 4 weeks after surgery and the transplants and surrounding tissue were evaluated by a histologic-histochemical grading scale, as described in Sellers et al., J. Bone Joint Surg., 79-A:1452-1463 (1997). Computerized image analysis of histologic sections was also performed. Results were evaluated using the unpaired Students t-test.

On gross examination, the joints showed no signs of inflammation. All the defects were filled by repair tissue. The surface appearance of the defects was variable but acceptable and did not correlate with form of treatment. Osteophytes were found in 3 joints (2 in the experimental group; 1 in control buffer group).

There was no correlation between the gross and histologic appearance in any of the defects. The presence of chondrocytes in the lacunae and sporadic cloning of cells in the donor cartilage indicated survival of the tissue. Focal degeneration of the donor cartilage was present in all of the control groups, but only one of the rhBMP-2 treated group. The healing of the defect in the rhBMP-2 treated group was significantly improved compared to that in the control group. The rhBMP-2 treated group had improved bony integration indicated by less fibrous repair tissue in the subchondral bone compartment. Treatment with rhBMP-2 also resulted in more cartilage above the original tidemark, apparently consisting of both donor tissue and newly regenerated recipient cartilage. There was no significant difference in the total amount of bone observed between the two groups.

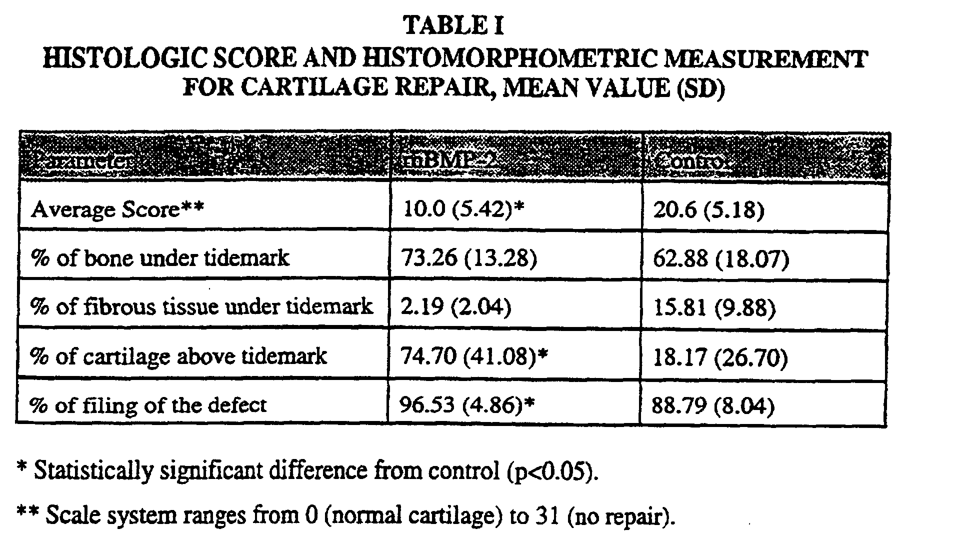

Additional histomorphometric analysis data further supports the beneficial effects of rhBMP-2 on the healing of graft. For example, the percentage filling of the new tissue above tide marker has been shown to be 81.52% in a rhBMP-2 treated group vs. 57.63% in control. There was less graft cartilage degeneration in rhBMP-2 treated group (23.83%) than in control group (44.52%). The integration of the graft or newly formed cartilage with the host cartilage was improved by rhBMP-2 treatment (56.48%) compared to that of control group (21.89%). More new cartilage formed under the influence of rhBMP-2 either at the edge of graft, which eliminated the gap between the graft and host, or at the top of graft, which made the graft more congruent with the joint surface.

The above results demonstrate that the healing of allogeneic osteochondral grafts in articular cartilage defects was improved by the addition of rhBMP-2. The active growth factor may have accelerated subchondral bone union, providing support and nutrition to the articular cartilage tissue. Addition of growth factor may also have stimulated new cartilage formation from recipient mesenchymal stem cells in the bone marrow and/or the synovial tissue. These results suggest that the combination of active growth factor, particularly the bone morphogenetic proteins, and osteochondral allografts might present a potent strategy for treatment of articular cartilage defects, particularly full thickness articular cartilage defects.

### II. Rabbit Autograft

Osteochondral grafts (2.7 mm in diameter and 3.0 mm long) were harvested from the trochlear groove or femoral condyle and transplanted into a donor site 2.7mm wide and 3.5mm long on the trochlear groove or femoral condyle of the knee joint in rabbits. Half the animals had buffer dripped into the recipient site prior to transplantation, and then the grafts were dipped in buffer for 2 minutes and placed into the recipient site. The other half had 5µg rhBMP-2 dripped into the recipient site prior to transplantation, and then the graft was dipped into buffer containing 500µg/ml rhBMP-2 for 2 minutes and then transplanted into the recipient site. The animals were sacrificed 4 weeks after surgery, and the recipient sites were evaluated histologically using both a histologic-histochemical grading scale [Sellers, et al., J. Bone Joint Surg., 79-A: 1452-63 (1997)] and quantitative computerized image analysis of the tissue. The data indicated that treatment with rhBMP-2 improved the healing of the autograft. The most dramatic effects were the reduction of graft cartilage degeneration (rhBMP-2 8.18% vs. control 36.25%), and more cartilage formed at the edge of graft (rhBMP-2 88.23% vs. control 50%).

### III. Non-Human Primate Autograft:

The non human primates used for autografts experiments were cynomologous macaques. Ostcochondral grafts (3.5mm diameter x 6mm long) were harvested from the trochlear groove of 6 cynomologous macaques and transplanted into recipient sites drilled into both the medial and lateral femoral condyle of the same animal (n=12 transplants total). Prior to transplantation 25µg rhBMP-2 was dripped into 6 recipient sites, and the grafts from those 6 transplants were dipped into a solution of 1.25mg/ml rhBMP-2 for 2 minutes. In the other 6 transplants, buffer alone was dripped into the recipient sites and the grafts were dipped into buffer alone for 2 minutes prior to transplantation. The limbs were immobilized in a cast for 2 weeks post-operatively, and the animals were sacrificed 9 weeks post operatively.

All the animals had normal function of their knee joints. On gross examination, the joints showed no signs of inflammation. Osteophytes were not found in any joint. Although the surface of the defects appeared level with the surrounding cartilage on gross examination, microscopic observation revealed subsidence of the grafts in most of the cases. The tissue observed grossly covering the surface was actually new-formed tissue on the top of graft. Computerized image analysis was performed by a blinded evaluator to quantitate percent filling of the defect, the new tissue types formed above the original tide mark, and the integration of the grafts and the surrounding cartilage. Favorable results were observed in the rhBMP-2 treated group in all these parameters. More new cartilage formed between the graft and host cartilage to eliminate the gap resulting in better integration of the graft with the surrounding cartilage (rhBMP-2 88.59% vs. control 64.82%). The filling of the cartilage defect was better in rhBMP-2 treated group (95.02%) than in the control group (86.68%). There was more fibrous tissue in the control group (11.90% vs. rhBMP-2 5.65%), while more transitional tissue was found in the rhBMP-2 treated group (36.38% vs. control 20.53%). There was no significant difference on the overall histologic-histochemical score between the two groups. Peripheral quantitative computered tomography (pQCT) showed that the bone density increased in the donor sites with time. At 6 weeks and 9 weeks after the operation, the tissue in the rhBMP-2 treated donor sites was significantly denser and the healing process was more advanced compared to control sites. Histologically, the donor sites contained regenerated bone trabeculae with fibrous tissue at the surface in all the cases.

### IV. rhBMP-2 Retention Ex Vivo:

Retention of rhBMP-2 in osteochondral graft with this technique was evaluated with the grafts from non-human primates. The graft was dipped in a mixture solution of ¹²⁵I labeled rhBMP-2 and unlabeled rhBMP-2. Results showed that the amount of rhBMP-2 absorbed to graft was proportional to the concentration of the protein, and the time of soaking. Other factors, which affect the retention of rhBMP-2, included the size of graft, and the presence of marrow elements between trabecular bone.

### V. rhBMP-2 Retention Time Course In Vivo:

The time course of rhBMP-2 retention in osteochondral graft was evaluated in rabbits. A mixture solution of ¹²⁵I labeled rhBMP-2 and unlabeled rhBMP-2, which contained 5 ug rhBMP-2 and 20 uCi ¹²⁵I, was loaded to the graft before implantation. The animals were scanned with γ-camera during the follow-up time for 22 days post-operatively. Compared to the time course of collagen sponge as a carrier, the half time of rhBMP-2 in osteochondral graft was increased from 1 day to 3 days. The radioactivity of 10% of the starting point was maintained from 11 days of collagen sponge to 22 days of graft.

### VI. Non-Human Primate Allografts:

Donor sites (3.5 mm wide x 6 mm long) were removed from the trochlear grooves of 12 adult cynomologous macaques and transplanted into 3.5 x 6mm recipient sites in the medial and lateral femoral condyles of unrelated individuals. Half of the transplants were soaked in 1.25 mg/ml rhBMP-2 for 2 minutes prior to transplantation, and half were soaked in buffer. The identical procedure was performed on the other limb 7 weeks after the first surgery. The limb was immobilized in a cast for 2 weeks post operatively after each surgery, and the animals were sacrificed 9 weeks after the second surgery for histologic analysis.

These results suggest that the combination of active growth factor, particularly the bone morphogenetic proteins, and osteochondral autografts might present a potent strategy for treatment of articular cartilage defects, particularly full thickness articular cartilage defects. In other embodiments BMP-2 may also be applied to frozen osteochondral allograft for treatment of focal articular cartilage defect.

The foregoing descriptions detail presently preferred embodiments of the present invention. Numerous modifications and variations in practice thereof are expected to occur to those skilled in the art upon consideration of these descriptions. Those modifications and variations are believed to be encompassed within the claims appended hereto.

## Claims

1. An osteochondral graft for administration to an area in need of articular cartilage regeneration having applied thereto an effective amount of bone morphogenetic protein (BMP) to stimulate osteogenic activity of infiltrating progenitor cells.

2. An osteochondral graft according to claim 1, wherein said graft is an allograft.

3. An osteochondral graft according to claim 1, wherein said graft is an autograft.

4. An osteochondral graft according to any one of claims 1 to 3, wherein about 0.05 to about 1.5 mg of BMP is applied thereto.

5. An osteochondral graft according to any one of claims 1 to 4, wherein said BMP is BMP-2.

6. An osteochondral graft according to any one of claims 1 to 5, having further applied thereto an effective amount of a protein which induces the formation of tendon or ligament tissue, wherein said protein is selected from the group consisting of BMP-12, BMP-13 and MP-52.

7. A process for preparing an osteochondral graft for administration to an area in need of regeneration of articular cartilage, wherein said process comprises applying to an osteochondral graft tissue source an effective amount of at least one purified bone morphogenetic protein (BMP) to stimulate osteogenic activity of infiltrating progenitor cells.

8. A process according to claim 7, wherein said tissue source is an allograft.

9. A process according to claim 7, wherein said tissue source is an autograft.

10. A process according to any one of claims 7 to 9, wherein step (ii) comprises applying to the tissue source from about 0.05 to about 1.5 mg of BMP.

11. A process according to any one of claims 7 to 10, wherein said BMP is selected from the group consisting of BMP-12, BMP-13 members of the BMP-12 subfamily and BMP-2.

12. A process according to any one of claims 7 to 10, wherein said BMP is BMP-2.

13. Use of at least one purified morphogenetic protein (BMP) in the preparation of a medicament for improving integration of an osteochondral graft when administered therewith to an area in need of articular cartilage regeneration.

## Patentansprüche

1. Osteochondrales Transplantat zur Verabreichung an einen Bereich mit Bedarf an artikulärer Knorpelregeneration, worauf eine wirksame Menge eines Knochen-morphogenen Proteins (BMP) aufgebracht ist, um osteogene Wirksamkeit von infiltrierenden Progenitorzellen zu stimulieren.

2. Osteochondrales Transplantat gemäß Anspruch 1, wobei besagtes Transplantat ein Allograft ist.

3. Osteochondrales Transplantat gemäß Anspruch 1, wobei besagtes Transplantat ein Autograft ist.

4. Osteochondrales Transplantat gemäß einem der Ansprüche 1 bis 3, wobei darauf etwa 0,05 bis etwa 1,5 mg BMP aufgebracht ist.

5. Osteochondrales Transplantat gemäß einem der Ansprüche 1 bis 4, wobei besagtes BMP BMP-2 ist.

6. Osteochondrales Transplantat gemäß einem der Ansprüche 1 bis 5, worauf ferner eine wirksame Menge eines Proteins aufgebracht ist, welches die Bildung von Tendo- oder Ligamentgewebe herbeiführt, wobei besagtes Protein ausgewählt wird aus der Gruppe bestehend aus BMP-12, BMP-13 und MP-52.

7. Verfahren zum Herstellen eines osteochondralen Transplantats zur Verabreichung an einen Bereich mit Bedarf an Regeneration von artikulärem Knorpel, wobei besagtes Verfahren Aufbringen einer wirksamen Menge von mindestens einem gereinigten Knochen-morphogenen Protein (BMP) auf eine osteochondrale Transplantat-Gewebequelle umfasst, um osteogene Wirksamkeit von infiltrierenden Progenitorzellen zu stimulieren.

8. Verfahren gemäß Anspruch 7, wobei besagte Gewebequelle ein Allograft ist.

9. Verfahren gemäß Anspruch 7, wobei besagte Gewebequelle ein Autograft ist.

10. Verfahren gemäß einem der Ansprüche 7 bis 9, wobei Schritt (ii) Aufbringen von etwa 0,05 bis etwa 1,5 mg BMP auf die Gewebequelle umfasst.

11. Verfahren gemäß einem der Ansprüche 7 bis 10, wobei besagtes BMP ausgewählt wird aus der Gruppe bestehend aus BMP-12, BMP-13, Mitgliedern der BMP-12 Unterfamilie und BMP-2.

12. Verfahren gemäß einem der Ansprüche 7 bis 10, wobei besagtes BMP BMP-2 ist.

13. Verwendung von mindestens einem gereinigten morphogenen Protein (BMP) bei der Herstellung eines Medikaments zum Verbessern der Integration eines osteochondralen Transplantats, wenn es damit an einen Bereich mit Bedarf an artikulärer Knorpelregeneration verabreicht wird.

## Revendications

1. Greffon ostéochondral destiné à être administré à une zone nécessitant une régénérescence du cartilage articulaire, sur laquelle a été appliquée une quantité efficace de protéine morphogénétique osseuse (BMP) en vue de stimuler l'activité ostéogénique de cellules souche d'infiltration.

2. Greffon ostéochondral selon la revendication 1, **caractérisé en ce que** ledit greffon est un allogreffon.

3. Greffon ostéochondral selon la revendication 1, **caractérisé en ce que** ledit greffon est un autogreffon.

4. Greffon ostéochondral selon l'une quelconque des revendications 1 à 3, **caractérisé en ce que** environ 0,05 à environ 1,5 mg de BMP sont appl iqués sur celui-ci.

5. Greffon ostéochondral selon l'une quelconque des revendications 1 à 4, **caractérisé en ce que** ladite BMP est la BMP-2.

6. Greffon ostéochondral ,selon l'une quelconque des revendications 1 à 5, sur lequel a été en outre appliquée une quantité efficace d'une protéine qui induit la formation d'un tendon ou d'un tissu de ligament, **caractérisé en ce que** ladite protéine est sélectionnée parmi le groupe constitué de BMP-12, BMP-13 et MP-52.

7. Procédé pour préparer un greffon ostéochondral destiné à être administré à une zone nécessitant une régénérescence du cartilage articulaire, **caractérisé en ce que** ledit procédé comprend l'application sur une source tissulaire de greffon ostéochondral d'une quantité efficace d'au moins une protéine morphogénétique osseuse (BMP) purifiée en vue de stimuler l'activité ostéogénique de cellules souche d'infiltration.

8. Procédé selon la revendication 7, **caractérisé en ce que** ladite source tissulaire est un allogreffon.

9. Procédé selon la revendication 7, **caractérisé en ce que** ladite source tissulaire est un autogreffon.

10. Procédé selon l'une quelconque des revendications 7 à 9, **caractérisé en ce que** l'étape (ii) comprend l'application sur la source tissulaire d'environ 0,05 à environ 1,5 mg de BMP.

11. Procédé selon l'une quelconque des revendications 7 à 10, **caractérisé en ce que** ladite BMP est sélectionnée parmi le groupe constitué des éléments BMP-12, BMP-13 de la sous-famille de BMP 12 et de la BMP-2.

12. Procédé selon l'une quelconque des revendications 7 à 10, **caractérisé en ce que** ladite BMP est la BMP-2.

13. Utilisation d'au moins une protéine morphogénétique (BMP) purifiée dans la préparation d'un médicament pour améliorer l'intégration d'un greffon ostéochondral lorsqu'il est administré avec celle-ci à une zone nécessitant une régénérescence du cartilage articulaire.
